# EUROPÄISCHE PATENTSCHRIFT

(11) **EP 2 004 259 B1**
(45) Veröffentlichungstag und Bekanntmachung des Hinweises auf die Patenterteilung: **30.05.2012**
(21) Anmeldenummer: 07711859.4
(22) Anmeldetag: 08.03.2007
(51) Int. Cl.: A61M 11/06

(54) **Inhalationstherapievorrichtung mit mehrfachen Düsen**
Inhalation therapy device with multiple nozzles
Dispositif de thérapie par inhalation muni de buses multiples

(30) Priorität: 11.04.2006 DE 102006017002
(43) Veröffentlichungstag der Anmeldung: 24.12.2008
(73) Patentinhaber: PARI GmbH Spezialisten für effektive Inhalation, 82319 Starnberg (DE)
(72) Erfinder: BOEHM, Andreas, 86934 Reichling (DE); LUBER, Martin, 82064 Strasslach Dingharting (DE); ROSENBEIGER, Sven, 82319 Starnberg (DE)
(74) Vertreter: HOFFMANN EITLE
(86) Internationale Anmeldenummer: PCT/EP2007/002030
(87) Internationale Veröffentlichungsnummer: WO 2007/118557

(56) Entgegenhaltungen:
- EP-A- 1 417 982
- DE-B- 1 147 355
- FR-A- 401 698

## Beschreibung

Die Erfindung betrifft eine Verneblervorrichtung für Inhalationstherapiegeräte zum Vernebeln eines Fluids, insbesondere einer therapeutisch wirksamen Flüssigkeit.

Im Stand der Technik sind seit langer Zeit Verneblerdüsen für Inhalationstherapiegeräte für das Vernebeln von flüssigen oder pulverförmigen Stoffen bekannt, bei denen - die Verhebelung mit Hilfe von Druckluft oder einem anderen der Verneblerdüse zugeführten Druckmittel erfolgt. Bereits DE-AS 1 147 355 beschreibt eine solche Verneblerdüse, wobei die offenbarte Verneblerdüse den grundsätzlichen Aufbau zeigt und einen zentral in der Düse angeordneten Druckmittelkanal und benachbart dazu angeordnete Ansaugkanäle aufweist. Wenn die zugeführte Druckluft aus der Austrittsöffnung des Druckmittelkanals austritt, wird die zu vernebelnde Flüssigkeit durch die Ansaugkanäle angesaugt und im Austrittsbereich der Druckluft vernebelt. Verneblerdüsen mit diesem grundsätzlichen Aufbau wurden im Laufe der Zeit weiterentwickelt und verfeinert und um zusätzliche Elemente ergänzt. Schon DE-AS 1 147 355 beschreibt zur Verbesserung der Verneblerdüse ein der Austrittsöffnung für das Druckmittel gegenüberliegendes Gasstromsteuer mit einer zu den Austrittsöffnungen hin keilförmigen Oberfläche. Maßnahmen, die die Effizienz der Verneblerdüse oder des die Verneblerdüse umfassenden Inhalationstherapiegeräts steigern, sind auch aus anderen Veröffentlichungen bekannt, wie zum Beispiel EP 0 170 715 A oder EP 0 261 649 A.

Dementsprechend liegen umfangreichen Erfahrungen hinsichtlich der Konstruktion dieser Düsen vor. Außerdem sind geeignete Druckgasquellen, beispielsweise tragbare Kompressoren entwickelt worden, die im Laufe der Zeit immer weiter optimiert wurden.

Die Erfindung strebt eine weitere Verbesserung und insbesondere eine Steigerung des Wirkungsgrades bei Düsenverneblern an.

Dieses Ziel wird erreicht durch eine Verneblervorrichtung mit den Merkmalen des Anspruchs 1, Vorteilhafte Ausgestaltungen ergeben sich aus den Unteransprüchen.

Im Folgenden wird die Erfindung anhand von Ausführungsbeispielen und unter Bezugnahme auf die Zeichnungen genauer erläutert. In den Zeichnungen zeigt
- Fig. 1: eine schematische und vereinfachte Ansicht eines Ausführungsbeispiels einer erfindungsgemäßen Verneblervorrichtung;
- Fig. 2: eine geschnittene Ansicht des Ausführungsbeispiels einer erfindungsgemäßen Verneblervorrichtung im Zusammenhang mit einem Fluidbehälter;
- Fig. 3A und 3B: eine vereinfachte Ansicht verschiedener Ausgestaltungen der Fluidaustrittsöffnungen;
- Fig. 4: eine Darstellung der Düsenpositionen zur Erläuterung der Abmessungen und Anordnung; und
- Fig. 5A und 5B: die Ausgestaltung eines Gasstromsteuers bzw. von Pralldornen.

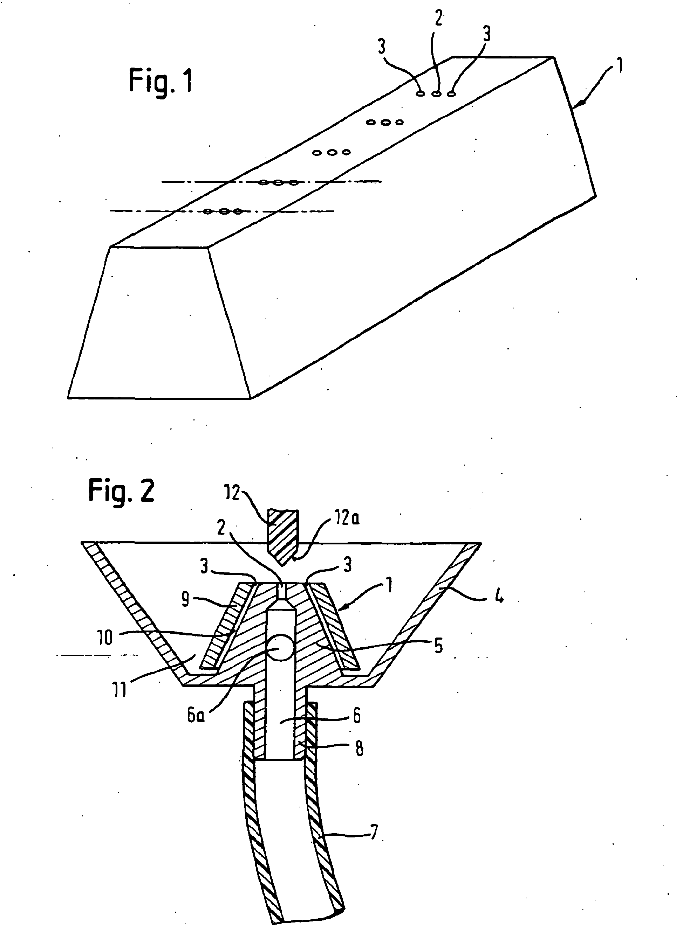

Figur 1 zeigt eine schematische und vereinfachte Ansicht einer Verneblervorrichtung 1 gemäß der Erfindung, wobei insbesondere die Anordnung der Druckgasaustrittsöffnungen 2 und der zugeordneten Fluidaustrittsöffnungen 3 dargestellt ist. Weitere Details, die in Figur 1 nicht gezeigt sind, ergeben sich aus der weiter unten folgenden Beschreibung der übrigen Figuren.

Wie in Figur 1 erkennbar ist, sind bei einer erfindungsgemäßen Verneblervorrichtung 1 mehrere, zumindest aber drei Öffnungen 2 vorhanden, aus denen ein der Vorrichtung zugeführtes Druckgas austreten kann und die in einer Reihe angeordnet sind. Deshalb besitzt die erfindungsgemäße Verneblervorrichtung 1 eine längliche Grundform, wie Figur 1 zu entnehmen ist. Zugeordnet zu jeder Druckgasaustrittsöffnung 2 sind jeweils zumindest zwei Öffnungen 3, aus denen ein zu vernebelndes Fluid austreten kann. Die Fluidaustrittsöffnungen 3 sind in dem in Figur 1 gezeigten Beispiel in einer Reihe mit der Druckgasaustrittsöffnung 2 angeordnet. Mit anderen Worten, bei dem in Figur 1 gezeigten Beispiel sind die Druckgasaustrittsöffnung 2 und die beiden Fluidaustrittsöffnungen 3 entlang einer Geraden angeordnet. Die Ausrichtung dieser Reihe ist vorzugsweise senkrecht zu der Reihe der Druckgasaustrittsöffnungen 2, wie auch Figur 1 zeigt.

Wenn aus den Druckgasaustrittsöffnungen 2 das zugeführte Druckgas austritt, wird ein zu den Fluidaustrittsöffnungen zugeführtes Fluid im Bereich vor den Austrittsöffnungen 2, 3 zu einem Aerosol vernebelt. Jede Druckgasaustrittsöffnung 2 bildet mit den ihr zugeordneten Fluidaustrittsöffnungen 3 quasi eine selbständige Verneblerdüse, wobei überraschenderweise die Effizienz der erfindungsgemäßen Verneblervorrichtung 1 mit ihren mehreren Verneblerdüsen 2, 3 insgesamt besser ist als die Effizienz einer einzelnen Verneblerdüse, die so ausgelegt ist, dass die Flächeninhalte der einzigen Druckgasaustrittsöffnung der Summe der Flächeninhalte der mehrfachen Druckgasaustrittsöffnungen 2 gemäß der Erfindung entsprechen. Dieser Aspekt soll im Folgenden anhand eines Berechnungsbeispiels verdeutlicht werden.

Bei einer typischen Verneblerdüse der bisher bekannten Art beträgt der Durchmesser der Druckgasaustrittsöffnung beispielsweise 0,48 mm, so dass sich eine Austrittsfläche für das Druckgas von 0,181 mm² ergibt. Ferner besitzt die Druckgasaustrittsöffnung einen Umfang von 1,51 mm. Bei einer erfindungsgemäßen Verneblervorrichtung besitzen die einzelnen Druckgasaustrittsöffnungen beispielsweise einen Durchmesser von 0,2 mm, so dass die Austrittsfläche 0,031 mm² und der Umfang der Austrittsöffnungen 0,63 mm beträgt. Um annähernd die gleiche Düsenfläche bereitzustellen, sind bei einer erfindungsgemäßen Verneblervorrichtung demnach ca. fünf (oder sechs) Einzeldüsen mit 0,2 mm Durchmesser in einer Reihe anzuordnen, entsprechend dem Verhältnis der Einzeldüsenfläche im Vergleich mit der Austrittsfläche einer herkömmlichen Düse; dieses Verhältnis liegt bei dem zuvor geschilderten Beispiel bei 5,84:1 (= 0,181/0,031). Durch die Beibehaltung des Flächeninhalts für den Austritt des Druckgases ergeben sich einige Vorteile. So kann auf Erfahrungen mit herkömmlichen Ein-Düsen-Verneblern zurückgegriffen und damit bei der Konstruktion bereits vorhandene Erfahrungswerte verwendet werden. Ferner kann eine erfindungsgemäße Verneblervorrichtung mit den herkömmlicherweise verwendeten Druckgasquellen, insbesondere den vorhandenen Kompressoren verwendet werden. Eine Anpassung der Kompressoren ist nicht erforderlich, da annähernd die gleiche Druckgasaustrittsfläche für den Austritt des Druckgases auch bei der erfindungsgemäßen Verneblervorrichtung zur Verfügung steht.

Trotz des annähernd angeglichenen Flächeninhalts der Druckgasaustrittsöffnungen 2 einer erfindungsgemäßen Verneblervorrichtung 1 führt die erfindungsgemäße Ausgestaltung der Düsenanordnung zu einer Erhöhung des Wirkungsgrades der Vernebelung. Dies wird erfindungsgemäß durch die Vergrößerung der Fluid/Druckgas-Grenzfläche, die im Wesentlichen durch den Umfang der Austrittsöffnungen 2 bestimmt wird, zwischen Düsenstrahl und zu vernebelnden Flüssigkeit bei gleich bleibender effektiver Düsenfläche durch eine erhöhte Anzahl an Düsen mit kleinerem Durchmesser erreicht. Bei gleich bleibenden effektiven Düsenflächen und deutlich höherer Grenzfläche ist der energetische Umsatz im Vergleich zu einem System mit einer einzelnen Düse zwar konstant, aber durch die erhöhte Grenzfläche wird die Effizienz gesteigert, das heißt es wird mehr Flüssigkeit vernebelt.

Wie bereits erwähnt, erlaubt die Angleichung der Summenfläche der Druckgasaustrittsöffnungen 2 im Vergleich zu herkömmlichen Düsen, dass vorhandene Kompressorsysteme ohne Modifikation auch mit einer erfindungsgemäßen Verneblervorrichtung 1 eingesetzt werden können. Aufgrund der gesteigerten Effizienz besteht darüber hinaus aber die Möglichkeit, eine Verneblervorrichtung mit einer kleineren Düsengesamtfläche zu realisieren, die mit einem entsprechend kleineren Kompressor betrieben werden kann und dabei dennoch die gleiche Effizienz wie ein herkömmliches System erreicht. Der kleinere Kompressor kann dann auch baulich verkleinert werden, so dass die Transportabilität des Therapiesystems, das im Wesentlichen aus dem Kompressor und dem Vernebler besteht, weiter gesteigert wird.

Figur 2 zeigt eine erfindungsgemäße Verneblervorrichtung 1 in einem Fluidbehälter 4 für die Aufnahme eines zu vernebelnden Fluids. In der geschnittenen Darstellung der Figur 2 ist erkennbar, dass die hier gezeigte Verneblervorrichtung 1 gemäß der Erfindung einen inneren Körper 5 aufweist, in dem die Druckgaskanäle 6 ausgebildet sind. Die Druckgaskanäle 6 dienen der Verteilung des Druckgases, das über einen Druckgasschlauch 7 zugeführt wird, der an einem Anschlussstutzen 8 der Vorrichtung angeschlossen ist. Die Druckgaskanäle 6 im inneren Körper 5 der erfindungsgemäßen Verneblervorrichtung 1 münden in den Druckgasaustrittsöffnungen 2, von denen in Figur 2 Aufgrund der geschnittenen Darstellung nur eine gezeigt ist. Der innere Körper 5 der erfindungsgemäßen Verneblervorrichtung 1 gemäß Figur 2 ist bei dem gezeigten Ausführungsbeispiel einstückig mit dem Fluidbehälter 4 und dem Anschlussstutzen 8 ausgebildet.

Auf den inneren Körper 5 der erfindungsgemäßen Verneblervorrichtung 1 ist ein äußerer Körper 9, beispielsweise eine Hülse oder eine Haube aufgesetzt, wobei in Figur 2 erkennbar ist, dass äußerer Körper 9 und innerer Körper 5 an konisch ausgebildeten Oberflächen miteinander in Kontakt stehen so dass sich der äußere Körper 9 auf dem inneren Körper 5 abstützt. Bei der Schnittdarstellung in Figur 2 sind Fluidkanäle 10 gezeigt, die in der Schnittebene liegen. Durch die Fluidkanäle 10 wird ein in dem Behälter bevorratetes Fluid den Fluidaustrittsöffnungen 3 zugeführt, wofür sich die Fluidkanäle 10 jeweils von der Fluidaustrittsöffnung 3 bis in den Bevorratungsbereich 11 des Behälters 4 erstrecken. Aufgrund dieser Ausgestaltung arbeitet die erfindungsgemäße Verneblervorrichtung 1 nach dem Venturi-Prinzip, da das aus der Druckgasaustrittsöffnung austretende Druckgas die in dem Behälter bevorratete Flüssigkeit durch die Fluidkanäle ansaugt, so dass die Flüssigkeit an den Fluidaustrittsöffnungen austritt und von dem austretenden Druckgas vernebelt wird.

In Figur 2 ist neben dem eine.Verlängerung des Anschlussstutzens bildenden Druckgaskanal 6 ein quer dazu verlaufender Druckgaskanal 6a erkennbar, der sich in Längsrichtung der erfindungsgemäßen Verneblervorrichtung 1 erstreckt, was der Anordnung der Düsenöffnungen 2 gemäß der Erfindung entspricht. Dieser Druckgaskanal 6a bewirkt, zusammen mit weiteren Druckgaskanälen, die zu den Druckgasaustrittsöffnungen 2 führen und die dem in Figur 2 gezeigten Druckgaskanalabschnitt entsprechen, eine Zuführung des Druckgases zu den multiplen Verneblerdüsen gemäß der Erfindung.

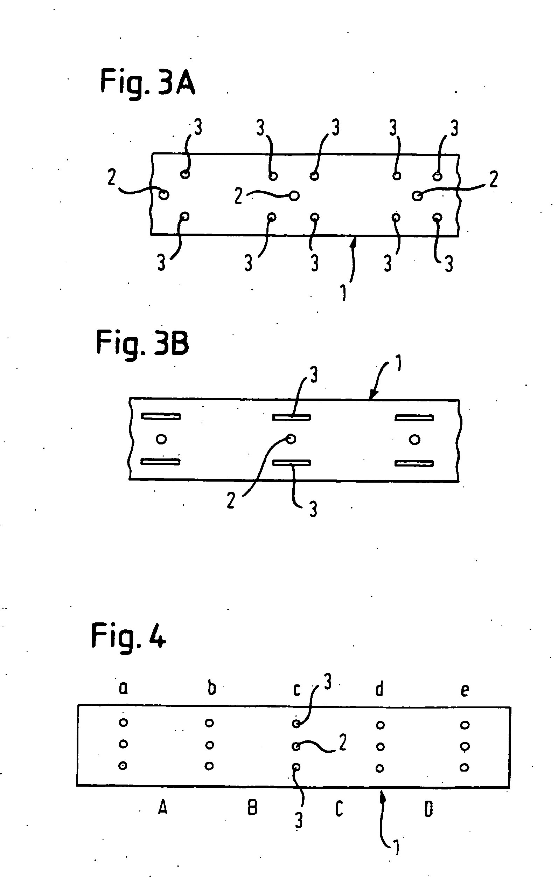

Figur 3A zeigt in einer vereinfachten Draufsicht eine erfindungsgemäße Verneblervorrichtung, bei der jeder Druckgasaustrittsöffnung 2 vier Fluidaustrittsöffnungen 3 zugeordnet sind. Die Anordnung ist bei diesem Ausführungsbeispiel der Erfindung diagonal über Kreuz in Bezug auf die Ausrichtung der Reihe der Druckgasaustrittsöffnungen 2. Auch bei dieser Anordnung bilden die Druckgasaustrittsöffnungen 2 gemeinsam mit den vier zugeordneten Fluidaustrittsöffnungen 3 jeweils eine selbständige Düse. Die Anzahl der Fluidaustrittsöffnungen 3 kann auch höher sein.

Figur 3B zeigt in einer vereinfachten Draufsicht eine Ausgestaltung der erfindungsgemäßen Verneblervorrichtung 1, bei der die Fluidaustrittsöffnungen 3 in Form von länglichen Schlitzen realisiert sind. Jeweils ein Schlitz ist auf beiden Seiten der Druckgasaustrittsöffnungen 2 angeordnet, wobei die Längsachse der Schlitze bei dem gezeigten Ausführungsbeispiel parallel zu der Geraden verläuft, an der entlang die Druckgasaustrittsöffnungen 2 aufgereiht sind. Die Länge der Schlitze ist in weiten Bereichen, frei wählbar, wobei aber bei längeren Schlitzen die Breite der Schlitze verringert werden muss, damit die einzelnen Düsen gemäß der Erfindung das Fluid effektiv vernebeln und beispielsweise nach dem Venturi-Prinzip arbeiten.

Figur 4 zeigt eine vereinfachte schematische Ansicht der Düsenöffnungen und dient der Erläuterung der Auswahl der Abmessungen der Druckgasaustrittsöffnungen 2. Die folgende Tabelle gibt Durchmesserwerte für die Druckgasaustrittsöffnungen jeweils an den Positionen a bis e an. Die Abstandwerte A bis D liegen in der Größenordnung einiger weniger bis zu mehreren Millimetern. Der genaue Wert ist im Einzelfall unter anderem in Abhängigkeit von der Anzahl der Fluidaustrittsöffnungen und deren Durchmesser zu bestimmen.

**Tabelle 1**

| Beispiel | a | b | c | d | e . |
|---|---|---|---|---|---|
| Nr. 1 | 0,2 mm | 0,2 mm | 0,2 mm | 0,2 mm | 0,2 mm |
| Nr. 2 | 0,15 mm | 0,2 mm | 0,2 mm | 0,2 mm | 0,15 mm |
| Nr. 3 | 0,1 mm | 0,15 mm | 0,25 mm | 0,15 mm | 0,1 mm |
| Nr. 4 | 0,1 mm | 0,1 mm | 0,3 mm | 0,1 mm | 0, 1 mm, |

Der Tabelle 1 ist zu entnehmen, dass gemäß einer vorteilhaften Ausgestaltung der Erfindung die Größe des Düsendurchmessers zur Mitte der Reihe der Druckgasaustrittsöffnungen 3 hin zunimmt, wenn die Düsen nicht den gleichen Durchmesser besitzen.

**Tabelle 2**

| Beispiel | a | b | c | d | e |
|---|---|---|---|---|---|
| Nr. 5 | 0,2 mm | 0,15 mm | 0,15 mm | 0,15 mm | 0,2 mm |
| Nr. 6 | 0,4 mm | 0,2 mm | 0,1 mm | 0,2 mm | 0,4 mm |
| Nr. 7 | 0,2 mm | 0,1 mm | 0,25 mm | 0,1 mm | 0,2 mm |
| Nr. 8 | 0,25 mm | 0,1 mm | 0,4 mm | 0,1 mm | 0,25 mm |

Der Tabelle 2 ist zu entnehmen, dass gemäß weiteren vorteilhaften Ausgestaltungen der Erfindung die Größe des Düsendurchmessers der Reihe der Druckgasaustrittsöffnungen 3 im Hinblick auf die angestrebte Zerstäubung des Fluids oder die Geometrie des Inhalationstherapiegeräts, in dem die erfindungsgemäße Verneblervorrichtung eingesetzt wird, in weiten Grenzen frei gestaltet werden kann.

Die in den Tabellen angegebenen Werte sind beispielhaft. Die Werte müssen im Einzelfall, zum Beispiel experimentell, ermittelt werden, wobei auch die Eigenschaften des zu vernebelnden Fluids berücksichtigt werden können.

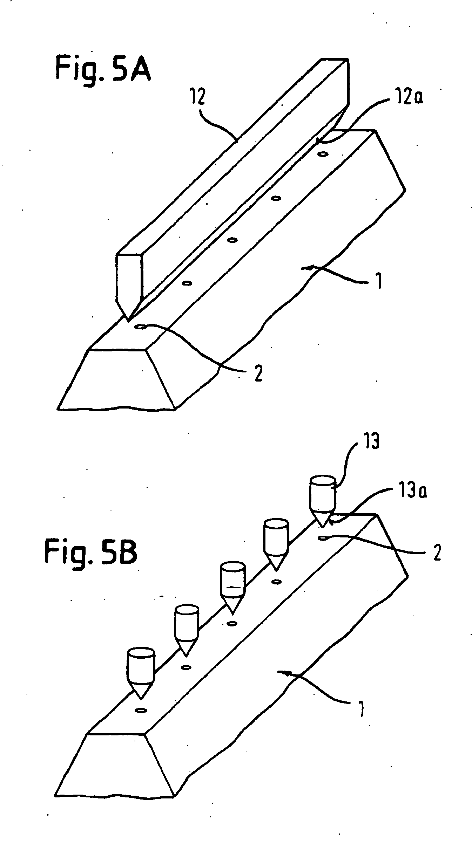

Wie in Figur 2 gezeigt, weist die erfindungsgemäße Verneblervorrichtung ein Gasstromsteuer 12 auf, das den Düsenöffnungen 2 gegenüberliegend angeordnet ist. In Anbetracht der länglich durch die Aufreihung der Düsenöffnungen bedingten Grundform der Verneblervorrichtung ist auch das Gasstromsteuer 12 länglich ausgebildet, wie Figur 5A zeigt. Auf der den Düsenöffnungen zugewandten Seite besitzt das Gasstromsteuer 12 vorteilhafterweise eine ebene Oberfläche, eine keilförmige Oberfläche 12a, siehe Figur 5A, eine abgerundete Oberfläche oder eine auf andere Weise geeignet gestaltete Oberfläche. Jedoch kann auch für jede einzelne Düse der erfindungsgemäßen Verneblervorrichtung 1 ein jeweils zugeordneter Pralldorn 13, siehe Figur 5B, vorgesehen werden, der der Druckgasaustrittsöffnung 2 gegenüberliegend angeordnet ist. Auf der der Öffnung zugewandten Seite besitzt der Pralldorn vorzugsweise eine angespitzte Oberfläche 13a, siehe Figur 5B, oder eine abgerundete Oberfläche.

## Patentansprüche

1. Verneblervorrichtung für Inhalationstherapiegeräte mit
einer Einrichtung (6, 6a) für die Zuführung eines unter Druck stehenden Gases, insbesondere Druckluft;
einer Einrichtung (10) für die Heranführung eines zu vernebelnden Fluids, insbesondere einer therapeutisch wirksamen Flüssigkeit;
mehreren Druckgasaustrittsöffnungen (2), aus denen ein zugeführtes Druckgas austritt; und
mehreren Fluidaustrittsöffnungen (3), aus denen das zu vernebelnde Fluid aufgrund des austretenden Druckgases austritt;
**dadurch gekennzeichnet, dass**
zumindest drei Druckgasaustrittsöffnungen (2) in einer Reihe angeordnet sind; und
jeweils einer der Druckgasaustrittsöffnungen (2) zumindest zwei Fluidaustrittsöffnungen (3) zugeordnet sind, die zusammen mit der zugeordneten Druckgasaustrittsäffnung eine Verneblerdüse bilden, und
die Druckgasaustrittsöffnungen (2) einen Durchmesser zwischen 0,1 und 0,4 mm aufweisen.

2. Verneblervorrichtung nach Anspruch 1, **dadurch gekennzeichnet, dass** die Druckgasaustrittsöffnungen (2) den gleichen Durchmesser aufweisen.

3. Verneblervorrichtung nach Anspruch 1, **dadurch gekennzeichnet, dass** die Druckgasaustrittsöffnungen (2) zumindest teilweise unterschiedliche Durchmesser aufweisen.

4. Verneblervorrichtung nach Anspruch 3, **dadurch gekennzeichnet, dass** die Druckgasaustrittsöffnungen (2) mit den größeren Durchmessern zur Mitte der Reihe von Druckgasaustrittsöffnungen hin angeordnet sind.

5. Verneblervorrichtung nach Anspruch 3, **dadurch gekennzeichnet, dass** die Druckgasaustrittsöffnungen (2) mit den größeren Durchmessern am Rand der Reihe von Druckgasaustrittsöffnungen hin angeordnet sind.

6. Verneblervorrichtung nach Anspruch 3, 4 oder 5, **dadurch gekennzeichnet, dass** die Durchmesser der Druckgasaustrittsöffnungen (2) der Reihe von Druckgasaustrittsöffnungen symmetrisch zur Mitte der Reihe angeordnet sind.

7. Verneblervorrichtung nach einem der vorangegangenen Ansprüche, **dadurch gekennzeichnet, dass** ein Gasstromsteuer (12) den Druckgasaustrittsöffnungen (2) gegenüberliegend vorgesehen ist.

8. Verneblervorrichtung nach Anspruch 6, **dadurch gekennzeichnet, dass** das Gasstromsteuer (12) eine den Druckgasaustrittsöffnungen zugewandte Oberfläche (12a) besitzt, die keilförmig oder abgerundet ist.

9. Verneblervorrichtung nach einem der vorangegangenen Ansprüche 1 bis 6, **dadurch gekennzeichnet, dass** für jede Druckgasaustrittsöffnung (2) ein Pralldorn (13) vorgesehen ist, der der Druckgasaustrittsöffnung (2) gegenüberliegend angeordnet ist.

10. Verneblervorrichtung nach Anspruch 9, **dadurch gekennzeichnet, dass** der Pralldorn (13) eine den Druckgasaustrittsöffnungen zugewandte Oberfläche (13a) besitzt, die angespitzt oder abgerundet ist.

11. Verneblervorrichtung nach einem der vorangegangenen Ansprüche, **dadurch gekennzeichnet, dass** die Fluidaustrittsöffnungen (3) kreisförmig sind.

12. Verneblervorrichtung nach einem der vorangegangenen Ansprüche 1 bis 11, **dadurch gekennzeichnet, dass** die Fluidaustrittsöffnungen (3) Schlitze sind.

13. Verneblervorrichtung nach Anspruch 12, **dadurch gekennzeichnet, dass** die Schlitze (3) sich parallel zur der Reihe der Druckgasaustrittsöffnungen (2) erstrecken.

14. Verneblervorrichtung nach einem der vorangegangenen Ansprüche, **dadurch gekennzeichnet, dass** zu den Fluidaustrittsöffnungen verlaufende Fluidkanäle (10) vorgesehen sind.

15. Verneblervorrichtung nach einem der vorangegangenen Ansprüche, **dadurch gekennzeichnet, dass** die Druckgasaustrittsöffnungen (2) einen Durchmesser kleiner 0,4 mm aufweisen.

16. Verneblervorrichtung nach Anspruch 15, **dadurch gekennzeichnet, dass** die Druckgasaustrittsöffnungen (2) einen Durchmesser zwischen 0,1 und 0,3 mm aufweisen.

## Claims

1. Nebuliser for inhalation therapy devices having
a device (6, 6a) for supplying a pressurised gas, in particular compressed air;
a device (10) for introducing a fluid to be nebulised, in particular a therapeutically effective liquid;
several compressed gas outlet openings (2), from which a supplied compressed gas emerges; and
several fluid outlet openings (3), from which the fluid to be nebulised emerges due to the emerging compressed gas,
**characterised in that**
at least three compressed gas outlet openings (2) are arranged in a row; and
at least two fluid outlet openings (3), which together with the assigned compressed gas outlet opening form a nebuliser nozzle, are assigned in each case to one of the compressed gas outlet openings (2), and
the compressed gas outlet openings (2) have a diameter between 0.1 and 0.4 mm.

2. Nebuliser according to claim 1, **characterised in that** the compressed gas outlet openings (2) have the same diameter.

3. Nebuliser according to claim 1, **characterised in that** the compressed gas outlet openings (2) have at least in some cases different diameters.

4. Nebuliser according to claim 3, **characterised in that** the compressed gas outlet openings (2) with the larger diameters are arranged towards the centre of the row of compressed gas outlet openings.

5. Nebuliser according to claim 3, **characterised in that** the compressed gas outlet openings (2) with the larger diameters are arranged towards the edge of the row of compressed gas outlet openings.

6. Nebuliser according to claim 3, 4 or 5, **characterised in that** the diameters of the compressed gas outlet openings (2) of the row of compressed gas outlet openings are arranged symmetrically to the centre of the row.

7. Nebuliser according to one of the preceding claims, **characterised in that** a gas flow control (12) is provided opposite the compressed gas outlet openings (2).

8. Nebuliser according to claim 6, **characterised in that** the gas flow control (12) has a surface (12a) facing the compressed gas outlet openings which is wedge-shaped or rounded.

9. Nebuliser according to one of the preceding claims 1 to 6, **characterised in that** a baffle rod (13), which is arranged opposite the compressed gas outlet opening (2), is provided for each compressed gas outlet opening (2).

10. Nebuliser according to claim 9, **characterised in that** the baffle rod (13) has a surface (13a) facing the compressed gas outlet openings which is pointed or rounded.

11. Nebuliser according to one of the preceding claims, **characterised in that** the fluid outlet openings (3) are circular.

12. Nebuliser according to one of the preceding claims 1 to 11, **characterised in that** the fluid outlet openings (3) are slots.

13. Nebuliser according to claim 12, **characterised in that** the slots (3) extend parallel to the row of compressed gas outlet openings (2).

14. Nebuliser according to one of the preceding claims, **characterised in that** fluid channels (10) running to the fluid outlet openings are provided.

15. Nebuliser according to one of the preceding claims, **characterised in that** the compressed gas outlet openings (2) have a diameter of less than 0.4 mm.

16. Nebuliser according to claim 15, **characterized in that** the compressed gas outlet openings (2) have a diameter of between 0.1 and 0.3 mm.

## Revendications

1. Système de nébulisation pour appareils de thérapie par inhalation, comportant
un dispositif (6, 6a) pour l'amenée d'un gaz sous pression, en particulier d'air comprimé ;
un dispositif (10) d'acheminement d'un fluide à nébuliser, en particulier d'un liquide à action thérapeutique ;
plusieurs ouvertures de sortie de gaz sous pression (2), dont sort un gaz sous pression acheminé ; et
plusieurs ouvertures de sortie de fluide (3), dont sort le fluide à nébuliser sous l'effet du gaz sous pression propulsé ;
**caractérisé**
**en ce qu'**au moins trois ouvertures de sortie de gaz sous pression (2) sont disposées sur une rangée ;
**en ce qu'**à chacune des ouvertures de sortie de gaz sous pression (2) sont associées au moins deux ouvertures de sortie de fluide (3), qui forment une buse de nébulisation avec l'ouverture de sortie de gaz sous pression associée, et
**en ce que** les ouvertures de sortie de gaz sous pression (2) ont un diamètre compris entre 0,1 et 0,4 mm.

2. Système de nébulisation selon la revendication 1, **caractérisé en ce que** les ouvertures de sortie de gaz sous pression (2) ont le même diamètre.

3. Système de nébulisation selon la revendication 1, **caractérisé en ce que** les ouvertures de sortie de gaz sous pression (2) ont des diamètres au moins partiellement différents.

4. Système de nébulisation selon la revendication 3, **caractérisé en ce que** les ouvertures de sortie de gaz sous pression (2) de diamètre supérieur sont disposées vers le milieu de la rangée des ouvertures de sortie de gaz sous pression.

5. Système de nébulisation selon la revendication 3, **caractérisé en ce que** les ouvertures de sortie de gaz sous pression (2) de diamètre supérieur sont disposées au bord de la rangée des ouvertures de sortie de gaz sous pression.

6. Système de nébulisation selon la revendication 3, la revendication 4 ou la revendication 5, **caractérisé en ce que** les diamètres des ouvertures de sortie de gaz sous pression (2) de la rangée des ouvertures de sortie de gaz sous pression sont ordonnés symétriquement par rapport au milieu de la rangée.

7. Système de nébulisation selon l'une des revendications précédentes, **caractérisé en ce qu'**un dispositif de commande du flux gazeux (12) est prévu en face des ouvertures de sortie de gaz sous pression (2).

8. Système de nébulisation selon la revendication 6, **caractérisé en ce que** le dispositif de commande du flux gazeux (12) présente une surface (12a) opposée aux ouvertures de sortie de gaz sous pression, laquelle est en forme de coin ou arrondie.

9. Système de nébulisation selon l'une des revendications 1 à 6, **caractérisé en ce qu'**un mandrin déflecteur (13) est prévu pour chaque ouverture de sortie de gaz sous pression (2), lequel est disposé en face de l'ouverture de sortie de gaz sous pression (2).

10. Système de nébulisation selon la revendication 9, **caractérisé en ce que** le mandrin déflecteur (13) présente une surface (13a) opposée aux ouvertures de sortie de gaz sous pression, laquelle est pointue ou arrondie.

11. Système de nébulisation selon l'une des revendications précédentes, **caractérisé en ce que** les ouvertures de sortie de fluide (3) sont circulaires.

12. Système de nébulisation selon l'une des revendications 1 à 11, **caractérisé en ce que** les ouvertures de sortie de fluide (3) sont des fentes.

13. Système de nébulisation selon la revendication 12, **caractérisé en ce que** les fentes (3) s'étendent parallèlement à la rangée des ouvertures de sortie de gaz sous pression (2).

14. Système de nébulisation selon l'une des revendications précédentes, **caractérisé en ce que** des canaux de fluide (10) s'étendant vers les ouvertures de sortie de fluide sont prévus.

15. Système de nébulisation selon l'une des revendications précédentes, **caractérisé en ce que** les ouvertures de sortie de gaz sous pression (2) ont un diamètre inférieur à 0,4 mm.

16. Système de nébulisation selon la revendication 15, **caractérisé en ce que** les ouvertures de sortie de gaz sous pression (2) ont un diamètre compris entre 0,1 et 0,3 mm.
